# EUROPÄISCHE PATENTSCHRIFT

(11) **EP 0 663 193 B1**
(45) Veröffentlichungstag und Bekanntmachung des Hinweises auf die Patenterteilung: **02.02.2000**
(21) Anmeldenummer: 93810891.7
(22) Anmeldetag: 20.12.1993
(51) Int. Cl.: A61F 2/34

(54) **Künstliche Hüftgelenkpfanne**
Artificial hip acetabular cup
Coque acétabulaire artificielle pour la hanche

(43) Veröffentlichungstag der Anmeldung: 19.07.1995
(73) Patentinhaber: Sulzer Orthopädie AG, 6340 Baar (CH)
(72) Erfinder: Wagner, Heinz, Prof. Dr. med., D-90592 Schwarzenbruck (DE); Roland, Willi, Ch-8413 Neftenbach (CH); Hermann, Breimesser, CH-8353 Elgg (CH)
(74) Vertreter: Heinen, Detlef

(56) Entgegenhaltungen:
- EP-A- 0 123 514
- EP-A- 0 205 132
- EP-A- 0 295 912
- EP-A- 0 445 068
- EP-A- 0 563 503
- FR-A- 2 684 544

## Beschreibung

Die Erfindung bezieht sich auf eine künstliche Hüftgelenkpfanne gemäss dem Oberbegriff von Anspruch 1. Die Erfindung bezieht sich weiter auf ein Verfahren zum Zusammenstellen sowie zum Ausrichten der erfindungsgemässen Hüftgelenkpfanne.

Aus der EP-A-0,123,514 ist eine künstliche Hüftgelenkpfanne bekannt, die eine Aussenschale mit einem äquatorialen Rand aufweist, in welche eine Innenschale einsetzbar ist, die eine kugelförmige Gestalt aufweist. Die Innenschale wird mit Hilfe eines Ringes fixiert, indem dieser Ring nach dem Ausrichten der Innenschale mittels Schrauben auf den Rand der Aussenschale aufgeschraubt wird und dadurch die Innenschale in der Aussenschale festhält.

Aus der EP-A-0563503 ist eine Stützvorrichtung für eine künstliche Hüftgelenkpfanne bekannt, die eine halbkugelförmige Stützschale umfasst, welche an ihrem äquatorialen Rand Stützlappen mit Durchbrechungen aufweist. Eine solche Stützschale eignet sich insbesondere dann als Implantat, wenn der Beckenknochen an der das Hüftgelenk tragenden Stelle beschädigt oder stark degeneriert ist. Der Beckenknochen weist insbesondere bei Reoperationen häufig einen solchen Zustand auf. Während dem Implantieren der bekannten Stützvorrichtung werden die Stützlappen durch plastische Deformation dem Verlauf des Beckenknochens angepasst um daraufhin die Stützvorrichtung mit durch die Stützlappen verlaufenden Knochenschrauben im Beckenknochen zu verankern. Daraufhin wird ein Knochenzement appliziert, der den Zwischenraum zwischen der Stützschale und dem Knochengewebe füllt und in der Stützschale ein Zementbett bildet, sodass eine Innenschale in der Stützschale positionierbar ist und durch den aushärtenden Knochenzement gehalten wird.

Der Nachteil einer Hüftgelenkpfanne, die die bekannte Stützvorrichtung und eine entsprechend angepasste Innenpfanne umfasst, ist darin zu sehen, dass zur Verankerung der gesamten Hüftgelenkpfanne ein Knochenzement erforderlich ist. Die Verwendung von Knochenzement weist bekanntlich mehrere Nachteile auf, unter anderem den, dass der Beckenknochen bei einer Reoperation in ungünstigen Fällen eine übermässige Schädigung erfahren kann.

Der Erfindung liegt die Aufgabe zugrunde, eine Hüftgelenkpfanne zu schaffen, die eine Stützvorrichtung mit Stützschale und flexiblen Stützlappen aufweist sowie eine Innenschale umfasst, die derart ausgestaltet sind, dass die Hüftgelenkpfanne ohne Knochenzement implantierbar ist.

Diese Aufgabe wird gelöst gemäss den Merkmalen von Anspruch 1. Die Unteransprüche beziehen sich auf weitere vorteilhafte Ausführungsformen. Die Erfindung wird weiter gelöst mit einem Verfahren zum Zusammenstellen einer erfindungsgemässen Hüftgelenkpfanne gemäss Anspruch 8.

Die erfindungsgemässe Hüftgelenkpfanne umfasst eine halbkugelförmige Stützschale, an deren äquatorialen Rand Stützlappen mit Durchbrechungen für Knochenschrauben angeordnet sind. Weiter umfasst die Hüftgelenkpfanne eine halbkugelförmige Innenschale, die in die entsprechend dimensionierte, halbkugelförmige Stützschale einsetzbar ist. Die Hüftgelenkpfanne umfasst weiter eine Haltevorrichtung, die vor der Öffnung des halbkugelförmigen Hohlraumes der Stützschale positionierbar ist, und mittels Befestigungsmittel fest mit der Stützschale verbindbar ist, sodass eine eingesetzte Innenschale unter Vorspannung zwischen der Stützschale und der Haltevorrichtung geklemmt und dadurch gehalten wird.

Die erfindungsgemässe Stützvorrichtung wird vorzugsweise mit durch die Stützlappen verlaufende Knochenschrauben im Beckenknochen verankert. Vor dem Befestigen der Stützvorrichtung wird diese vorerst derart an den Beckenknochen angelegt und die Stützlappen durch plastische Deformation dem Verlauf des Beckenknochens angepasst, dass die Stützlappen eine Auflage auf dem Beckenknochen finden, und dass das darunter liegende Knochenmaterial sich zur Verankerung mit Knochenschrauben eignet. Daraufhin wird die angepasste Stützvorrichtung entweder mit Knochenschrauben am Beckenknochen befestigt und daraufhin die gesamte Hüftgelenkpfanne zusammengestellt, oder die angepasste Stützvorrichtung wird nochmals herausgenommen, die gesamte Hüftgelenkpfanne zusammengestellt, und diese mit durch die Stützlappen verlaufende Knochenschrauben am Beckenknochen befestigt.

Ein Vorteil der Erfindung ist darin zu sehen, dass die Stützvorrichtung sehr elastisch ausgebildet sein kann, und die Stützvorrichtung somit mit geringem Kraftaufwand dem Verlauf des Beckenknochens anpassbar ist. Durch das Einspannen der Innenschale zwischen die Haltevorrichtung und die Stützvorrichtung erhält die gesamte Hüftgelenkpfanne eine genügend grosse Steifigkeit. Ein weiterer Vorteil ist somit darin zu sehen, dass die Hüftgelenkpfanne ohne Knochenzement fest mit dem Beckenknochen verankerbar ist.

In einer besonders vorteilhaften Ausführungsform der Hüftgelenkpfanne ist die Aussenfläche der Innenschale kugelförmig ausgebildet, und die Haltevorrichtung weist eine kreisförmige Ausnehmung mit vorstehenden Zähnen auf, sodass die Lage der Innenschale frei in der Stützschale positionierbar ist, und daraufhin die kreisförmige Ausnehmung der Haltevorrichtung auf die Aussenfläche der Innenschale gelegt wird und die Haltevorrichtung an der Stützvorrichtung befestigt wird, sodass die vorstehenden Zähne in der Aussenfläche eingreifen und die Stützvorrichtung und die Innenschale in einer gegenseitig definierten Lage fixiert sind. Der Vorteil dieser Ausführungsform ist darin zu sehen, dass die Stellung der Innenschale, zum Beispiel bei bereits befestigter Stützvorrichtung sehr einfach in eine orthopädisch günstige Lage positionierbar ist, und anschliessend mit der Haltevorrichtung befestigbar ist. Es kann sich als vorteilhaft erweisen, die Innenfläche der Stützschale mit zusätzlichen Eindringelementen wie Zähnen zu versehen, die während dem Einsetzen der Innenschale in die Aussenfläche der Innenschale eindringen, um ein Verdrehen der Innenschale zusätzlich zu verhindern.

Ein weiterer Vorteil der Erfindung ist darin zu sehen, dass die Befestigungsmittel der Haltevorrichtung zum Beispiel als ein Widerlager und als eine Einrastvorrichtung ausgestaltbar sind. Dabei wird die Haltevorrichtung vorerst derart mit der Stützvorrichtung in Wirkverbindung gebracht, dass die Verbindung ein Widerlager bildet. Danach wir die Haltevorrichtung in einer durch das Widerlager festgelegte Richtung zur Stützvorrichtung hin geschwenkt und durch eine an der Stützvorrichtung einrastenden Einrastvorrichtung fest mit der Stützvorrichtung verbunden. Die Haltevorrichtung lässt sich somit auf sehr einfache Weise mit der Stützvorrichtung verbinden.

Im folgenden wird die Erfindung anhand von Ausführungsbeispielen beschrieben. Es zeigen:
- Fig. 1: Einen Längsschnitt durch eine künstliche Hüftgelenkpfanne entlang der Linie (A-A);
- Fig. 2a: eine Untenansicht einer künstlichen Hüftgelenkpfanne mit einer schwenkbaren Haltevorrichtung;
- Fig. 2b: eine Untenansicht einer Hüftgelenkpfanne mit einer einrastbaren Haltevorrichtung;
- Fig. 2c: eine Untenansicht einer Hüftgelenkpfanne mit einer verschraubbaren Haltevorrichtung;
- Fig. 3a: eine Aufsicht sowie eine Seitenansicht einer Einrastvorrichtung;
- Fig. 3b: eine Aufsicht sowie eine Seitenansicht einer weiteren Einrastvorrichtung;
- Fig. 3c: eine Aufsicht sowie eine Seitenansicht einer weiteren Einrastvorrichtung
- Fig. 3d: eine perspektivische Ansicht einer weiteren Einrastvorrichtung;
- Fig. 4a: eine Seitenansicht einer Einrastvorrichtung;
- Fig. 4b: eine Aufsicht der Einrastvorrichtung gemäss Fig. 4a;
- Fig. 5a: eine Aufsicht eines Stützlappens mit einer Ausnehmung für ein Widerlager;
- Fig. 5b: eine Seitenansicht eines Befestigungsteil einer Haltevorrichtung, passend zum Widerlager gemäss Fig. 5a;
- Fig. 6a: eine Aufsicht einer weiteren Ausführungsform eines Stützlappens mit Widerlager;
- Fig. 6b: eine Seitenansicht eines weiteren Befestigungsteil einer Haltevorrichtung, passend zum Widerlager gemäss Fig. 6a.

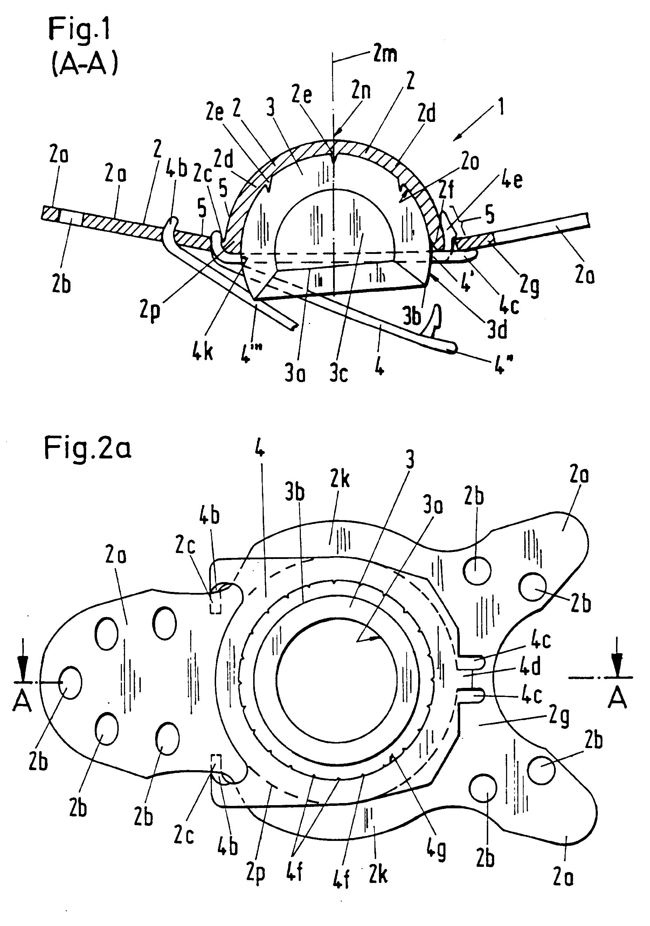

Fig. 2a zeigt eine Untenansicht eines Ausführungsbeispieles einer erfindungsgemässen Hüftgelenkpfanne, wobei der Schnitt (A-A) in Fig. 1 als einer Seitenansicht dargestellt ist.

Fig. 1 zeigt eine Hüftgelenkpfanne 1, die eine Stützvorrichtung 2, eine Innenschale 3, eine Haltevorrichtung 4, sowie Befestigungselemente 5 umfasst. Die Stützvorrichtug 2 besteht aus einer halbkugelförmigen Stützschale 2d, an deren äquatorialem Rand 2p Stützlappen 2a angeordnet sind. Die Stützlappen 2a weisen Durchbrechungen 2b auf zur Aufnahme von Knochenschrauben, die im Beckenknochen zu befestigen sind. Im vorliegenden Ausführungsbeispiel ist der äquatoriale Rand 2p von einem vorstehenden Rand 2g, 2k umgeben, der in die Stützlappen 2a mündet. Die Stützschale 2d weist eine halbkugelförmige Innenfläche 2o auf mit einem Pol 2n und einer Achse 2m. Die Stützschale 2d kann weitere Durchbrechungen aufweisen, z.B. für Knochenschrauben oder für Kontrollöffnungen. Weiter kann an der in ihrer Grundform als Halbkugel ausgebildeten Stützschale 2d im Polbereich eine Kalotte abgeschnitten sein, so dass die Stützschale 2d am Pol 2n eine grössere Oeffnungen aufweisen kann. Die Stützvorrichtung 2 besteht aus einem metallischen Material. Die Innenschale 3 dient zur Aufnahme eines Gelenkkugelkopfes und ist üblicherweise aus einem Kunststoff, z.B. aus Polyäthylen gefertigt. Die Innenschale kann jedoch auch aus Metall, z.B. aus Titan oder aus einem Porzellan gefertigt sein. Die Innenschale 3 weist einen Innenraum 3c auf, der halbkugelförmige ausgebildet ist, um den Kugelgelenkkopf gelenkig zu lagern. Der Innenraum 3c weist eine kreisförmige Oeffnung 3a auf, die sich gegen aussen zu einer äusseren Oeffnung 3b weitet. Die Aussenfläche 3d der Innenschale ist der Innenfläche 2o der Stützschale angepasst, wobei die Form der Aussenfläche 3d vorteilhafterweise kugelförmig ausgebildet ist. Die Innenfläche 2o der Stützschale 2d kann Eindringelemente 2e, z.B. Dornen, aufweisen, die bei eingesetzter Innenschale 3 in deren Aussenfläche 3d eindringen, um eine Relativbewegung zwischen Innenschale 3 und Stützvorrichtung 2 zu verhindern. Eine Haltevorrichtung 4 dient dazu, die Innenschale 3 fest in der Stützschale 2d zu verankern. Dazu wird die Haltevorrichtung 4 bei eingesetzter Innenschale 3 derart mit Befestigungsmittel 5 an der Stützvorrichtung 2 befestigt, dass die Oeffnung 3a, 3b nicht abgedeckt wird und derart, dass die Haltevorrichtung 4 eine Wirkverbindung zur Innenschale 3 aufweist. Im vorliegenden Ausführungsbeispiel ist das Befestigungsmittel 5 auf der einen Seite als Widerlager ausgebildet, auf der anderen Seite als Einrastvorrichtung 4d. Im vorliegenden Ausführungsbeispiel ist die Haltevorrichtung 4 in ihrem mittleren Bereich als ringförmiges Element ausgbildet, das eine kreisförmige Oeffnung 4g aufweist mit gegen den Innenraum vorstehenden Zähnen 4f. An diesem ringförmigen Teil ist auf der einen Seite als Verbindungsmittel eine Einrastvorrichtung 4d angeordnet, deren Eindringtiefe durch die Auflageelemente 4c begrenzt ist. Weiter sind an dem kreisförmigen Mittelteil zwei Abstützelemente 4b angeordnet derart, dass die beiden Abstützelemente 4b die Lappen 2a beidseitig umfassen. Die Stützvorrichtung 2 weist im Bereich des äquatorialen Randes 2p ein Auflager bzw. eine Auflagefläche 2c auf, mit denen die Abstützelemente 4d der Haltevorrichtung 4 in Wirkverbindung gebracht werden können, so dass sich zwischen den Abstützelementen 4b und der Auflagefläche 2c ein Widerlager eribt. Die Ausbildung der beiden Abstützelemente 4b erfordert es, dass die Haltevorrichtung 4 am Lappen 2a eingefädelt werden muss und die Haltevorrichtung 4 entlang dem Lappen 2a in ihre endgültige Position gebracht werden kann. Mit 4''' ist eine Lage einer Haltevorrichtung 4 dargestellt wie sie während des Einfädelvorganges auftreten kann.

Die erfindungsgemässe Hüftgelenkpfanne 1 wird nach dem folgenden Verfahren implantiert. In einem ersten Schritt wird für die metallische Stützvorrichtung 2 eine vorteilhafte Lage am Beckenknochen gesucht, die sich insbesondere dadurch auszeichnet, dass die Stützlappen 2a derart positionierbar sind, dass sie auf dem Beckenknochen aufliegen und dass der sich darunter befindliche Beckenknochen für die Aufnahme von Knochenschrauben eignet. Somit wird die metallische Stützvorrichtung 2 in einer vorteilhaften Lage am Beckenknochen positioniert und daraufhin die Stützlappen 2a plastisch verformt, um deren Verlauf dem Verlauf des Beckenknochens anzupassen. Daraufhin wird die Stützvorrichtung 2 vom Beckenknochen abgehoben, herausgenommen und die Haltevorrichtung 4 in den Stützlappen 2 eingefädelt. Daraufhin wird die Stützvorrichtung wieder in die vorgesehene Lage eingesetzt und mit Knochenschrauben, die durch die Durchbrechnungen 2b verlaufen, am Beckenknochen fixiert. Daraufhin wird die Innenschale 3 in die Stützschale 2d eingepasst. Da die sich gegenseitig berührenden Oberflächen der beiden Schale kugelförmige ausgebildet sind, lässt sich die Innenschale 3 in weiten Grenzen gegenüber der Stützschale 2d abkippen sowie gegenseitig verdrehen. Die Innenschale 3 weist eine kardanische Beweglichkeit in der Stützschale 2d auf. Bei der Positionierung der Stützschale 2 wurde insbesondere der Zustand des Beckenknochens berücksichtigt, um eine vorteilhafte Befestigung der Stützvorrichtung 2 mittels Knochenschrauben zu erreichen. Die kardanische Beweglichkeit der Innenschale 3 ermöglicht es, beim Einsetzen der Innenschale 3 diese in einer orthopädisch vorteilhaften Position zu fixieren. Im vorliegenden Ausführungsbeispiel weist die Innenfläche 2o der Stützschale 2d Eindringelemente 2e auf, so dass die Innenschale 3 in die Stützschale 2 eingepresst werden muss, damit die Eindringelemente 2e in die Aussenfläche 3d der Innenschale 3 eindringen. Dazu kann ein geeignetes Setzinstrument vorteilhaft sein, um bei vorgegebener Lage der Innenschale 3 diese unter Krafteinwirkung in die Stützschale 2d zu pressen. Daraufhin wird - wie in Fig. 1 dargetellt - die schwenkbare Haltevorrichtung 4 in die Position 4'' geschwenkt und weiter in die endgültige Position 4' geschwenkt, wobei die Haltevorrichtung 4 auf der einen Seite ein Widerlager 5 bildet und auf der anderen Seite eine Einrastvorrichtung 4d mit einem Einrastelement 4e, das durch eine Durchbrechung 2f im Bereich des äquatorialen Randes 2g hindurchtritt und an der dem Beckenknochen zugewandten Oberfläche der Stützvorrichtung 2 einrastet. Die Haltevorrichtung 4 weist eine kreisförmige Ausnehmung 4g mit Zähnen 4f auf, die sich kurz vor dem Einrasten der Haltevorrichtung kreisförmig um die Aussenflächen 3d der Innenschale 3 legen, so dass die Zähne 4f beim weiteren Einrastvorgang in die Oberfläche 3d der Innenschale 3 eindringen und ein Verdrehen der Innenschale 3 in Umfangsrichtung 4g verhindern. Dabei erfährt die Innenschale 3 eine in Richtung des Poles 2n wirkende Spannkraft, so dass die Innenschale 3 bei eingerasteter Haltevorrichtung 4 unter Vorspannung zwischen der Innenfläche 2o der Stützschale 2d und der Haltevorrichtung 4 gehalten ist. Die Innenschale ist somit gegen eine Verdrehung in Umfangsrichtung sowie gegen eine Bewegung in axialer Richtung 2m gesichert. Die dargestellte Haltevorrichtung 4, deren Befestigungsmittel 5 aus einem Widerlager sowie aus einer Einrastvorrichtung 4d besteht, lässt sich somit auf einfache Weise mit einer einzigen Schwenkbewegung fest mit der Stützvorrichtung 2 verbinden. Ein Vorteil des Widerlagers ist darin zu sehen, dass mit der Haltevorrichtung 4 eine Hebelwirkung erzielbar ist, die dazu dienen kann, die Innenschale 3 ohne ein Setzinstrument in die Stützschale 2d zu pressen.

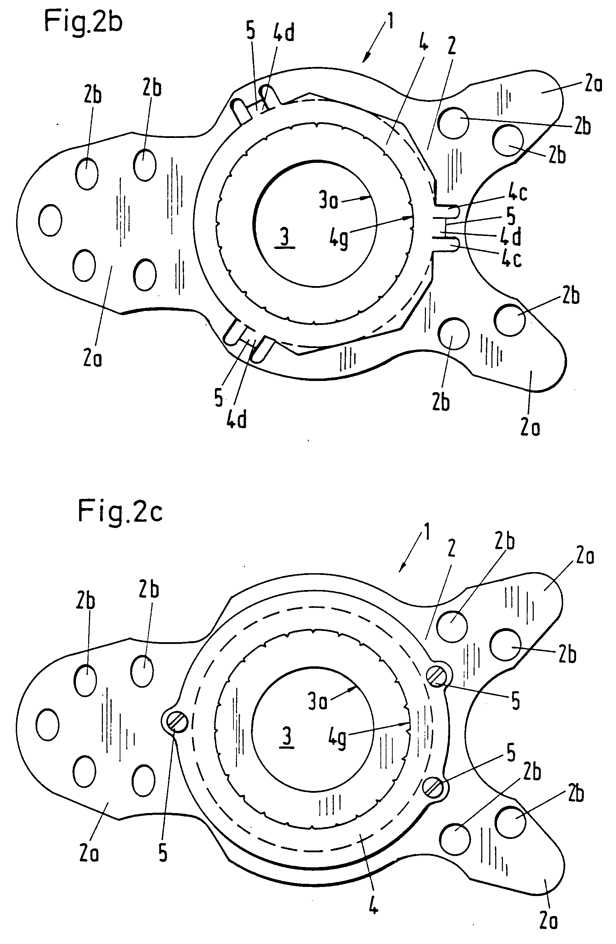

Fig. 2b zeigt eine weitere Untenansicht einer erfindungsgemässen Hüftgelenkpfanne 1, die eine metallische Stützvorrichtung 2, eine Innenschale 3 sowie eine Haltevorrichtung 4 und Verbindungsmittel 5 umfasst. Der Aufbau der Verbindungsmittel 5 ist identisch zu der in Fig. 1 dargestellten Einrastvorrichtung, so dass aus der in Fig. 2b dargestellten Untenansicht von jedem Befestigungsmittel 5 das Auflageelement 4c sowie ein Teil der Einrastvorrichtung 4d sichtbar ist. Nach dem Einfügen der Innenschale 3 in die Stützschale 2d wird die Haltevorrichtung 4 über die drei dargestellten Einrastvorrichtungen 4d an der Stützvorrichtung 2 eingerastet und befestigt. Das Einrasten kann gleichzeitig erfolgen oder, wie in Fig. 1 dargestellt, durch eine Schwenkbewegung der Haltevorrichtung 4, indem die Haltevorrichtung 4 mit ein oder zwei Einrastvorrichtungen 4d an der Stützvorrichtung 2 befestigt wird und die dritte Einrastvorrichtung 4d durch eine anschliessende Schwenkbewegung der Haltevorrichtung 4 eingerastet wird.

Fig. 2c zeigt ein weiteres Ausführungsbeispiel einer erfindungsgemässen Hüftgelenkpfanne 1, bestehend aus einer Stützvorrichtung 2, einer Innenschale 3, einer Haltevorrichtung 4 sowie Befestigungsmittel 5. Im dargestellten Ausführungsbeispiel sind die Befestigungsmittel 5 als Schrauben ausgeführt, deren Köpfe auf der Haltevorrichtung 4 aufliegen und deren Gewinde in eine in der Stützvorrichtung 2 angebrachten Bohrung eingreift. Nach dem Einfügen der Innenschale 3 in die Stützvorrichtung 2 wird die Haltevorrichtung 4 positioniert und mit den Schrauben 5 an der Stützvorrichtung 2 befestigt.

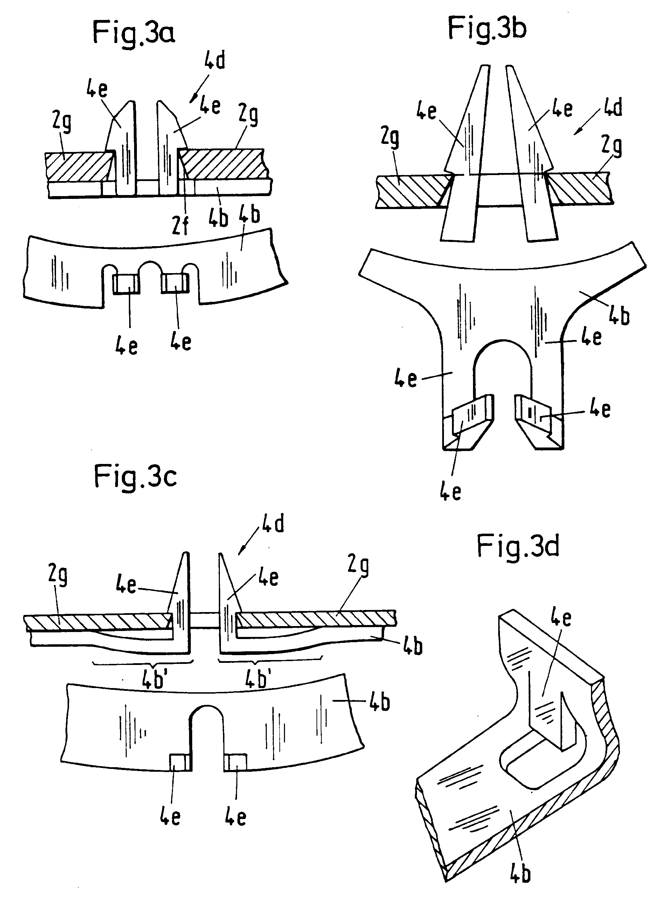

Die Fig. 3a bis 3d zeigen verschiedene Ausführungsformen von Einrastvorrichtungen. Fig. 3a zeigt in der Seitenansicht den vorstehenden Rand 2g der Stützvorrichtung 2 mit einer Durchbrechung 2f für die Einrastvorrichtung 4d, deren Eindringtiefe durch das Abstützelement 4b der Haltevorrichtung 4 begrenzt ist. Die Einrastelemente 4e sind fest am vorstehenden Rand 2g der Stützvorrichtung 2 eingerastet. Die Schenkelläge der Einrastelemente 4e ist relativ kurz ausgestaltet, was dem Einrastelement 4e eine hohe Steifigkeit verleiht. Die in Fig. 3a dargestellte Einrastvorrichtung 4d ergibt somit eine während des Einrastvorganges eher harte Verbindung, die nachträglich schwerer zu lösen ist. Fig. 3b zeigt eine weitere Einrastvorrichtung 4d mit relativ langschenkligen Einrastelementen 4e, was zur Folge hat, dass die Einrastelmenente 4e entgegen der wirkenden Federkraft leichter beweglich sind, d.h. elastischer sind, so dass diese Einrastvorrichtung 4d relativ einfach wieder geöffnet werden kann. Fig. 3c zeigt ein weiteres Ausführungsbeispiel einer Einrastvorrichtung 4d, wobei das Abstützelement 4b der Haltevorrichtung 4 im Bereich der Wirkverbindung 4b' vom vorstehenden Rand der Stützvorrichtung 2g abgehoben ist, so dass die eingerasteten Einrastelemente 4e eine Zugkraft vertikal zur Stützvorrichtung 2g erfahren.

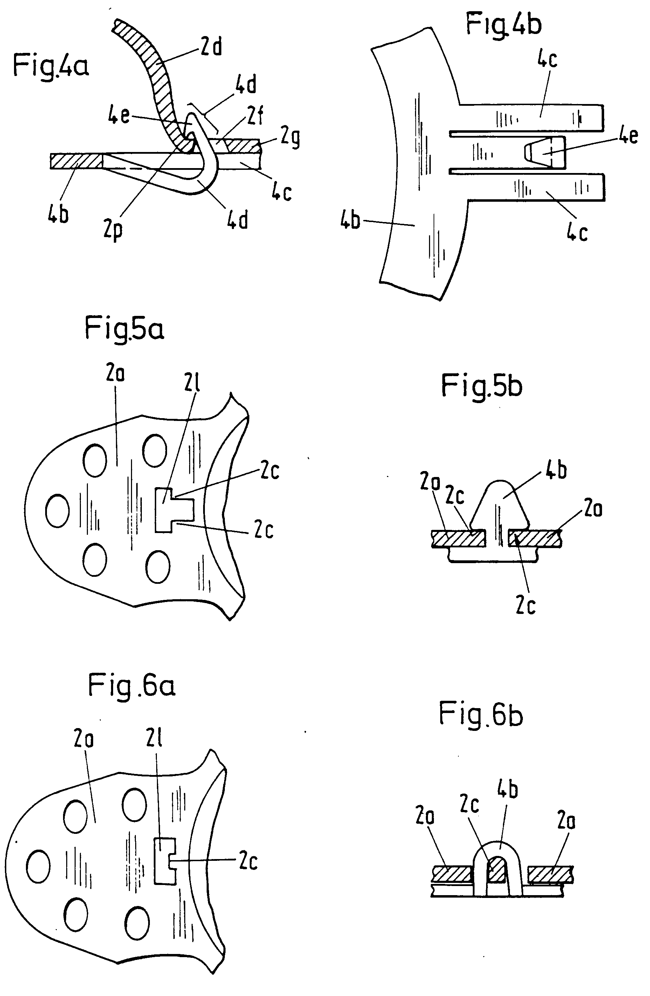

Fig. 4a zeigt eine weitere Ausgestaltung einer Einrastvorrichtung 4d. Die Stützvorrichtung 2 weist im Bereich des vorstehenden Randes 2g, unmittelbar anschliessend am äquatorialen Rand 2p, eine Durchbrechung 2f auf, die zum Durchtritt des Einrastelementes 4e dient. Die Haltevorrichtung 4 liegt mit dem Auflageelement 4c auf der Unterseite des vorstehenden Randes 2g auf, das Einrastelement 4e tritt durch die Ausnehmung 2f und rastet im Bereich des äquatorialen Randes 2p an der Aussenfläche der Stützschale 2d ein.

Fig. 4b zeigt eine Aufsicht der in Fig. 4a dargestellten Einrastvorrichtung.

Fig. 3d zeigt ein weiteres Ausführungsbeispiel eines Einrastelementes 4e mit Abstützteil 4b, das an Stelle der in Fig. 4a dargestellten Einrastvorrichtung 4d die Haltefunktion übernehmen könnte.

Die Fig. 5a, 5b, 6a, 6b zeigen zwei weitere Ausführungsbeispiele eines Widerlagers. Fig. 5a zeigt den Lappen 2a der Stützvorrichtung 2, der eine T-förmige Durchbrechung 2l mit zwei Auflageflächen 2c aufweist. Fig. 5b zeigt das in die Durchbrechnung 2l des Lappens 2a eingeführte Abstützteil 4b der Haltevorrichtung 4, das mit der Auflagefläche 2c in Wirkverbindung tretend ein Widerlager bildet. Fig. 6a zeigt den Lappen 2a einer Stützvorrichtung 2, der eine U-förmige Durchbrechung 2l mit einer Auflagefläche 2c aufweist. In Fig. 6b ist ein ringförmiges Abstützteil 4b der Haltevorrichtung 4 dargestellt, das auf der Auflagefläche 2c aufliegt und durch diese Wirkverbindung ein Widerlager bildet.

## Patentansprüche

1. Künstliche Hüftgelenkpfanne (1), umfassend
- eine metallische Stützvorrichtung (2), die aus einer Stützschale (2d) mit einem äquatorialen Rand (2p) sowie vom äquatorialen Rand (2p) nach aussen abstehenden Stützlappen (2a) besteht, welche Durchbrechungen (2b) für Knochenschrauben aufweisen,
- eine Innenschale (3) zur Aufnahme eines Gelenkkugelkopfes, welche in die Stützschale (2d) eingesetzt ist,
- eine Haltevorrichtung (4) und mindestens zwei Befestigungsmittel (5) umfasst, wobei die Haltevorrichtung (4) durch die Befestigungsmittel (5) fest mit der Stützvorrichtung (2) verbindbar ist, um die Innenschale (3) zwischen der Stützschale (2d) und der Haltevorrichtung (4) einzuspannen,
dadurch gekennzeichnet,
dass die Haltevorrichtung (4) ein Abstützelement (4b) umfasst und die Stützvorrichtung (2) eine Auflage (2c) aufweist, sodass sich bei einer Wirkverbindung zwischen dem Abstützelement (4b) und der Auflage (2c) ein Widerlager ergibt.

2. Künstliche Hüftgelenkpfanne (1) nach Anspruch 1, dadurch gekennzeichnet, dass die Haltevorrichtung (4) zwei symmetrisch angeordnete Abstützelemente (4b) aufweist, die einen Lappen (2a) beidseitig im wesentlichen umfassen und derart ein Befestigungsmittel (5) bilden.

3. Künstliche Hüftgelenkpfanne (1) nach einem der Ansprüche 1 oder 2, dadurch gekennzeichnet, dass die Innenschale (3) eine Aussenfläche (3d) aufweist, die kugelförmig ausgebildet ist, dass die Haltevorrichtung (4) eine kreisförmige Ausnehmung (4g) aufweist, und dass bei an der Stützvorrichtung (2) befestigter Haltevorrichtung (4) der Rand der kreisförmigen Ausnehmung (4g) auf der Aussenfläche (3d) aufliegt.

4. Künstliche Hüftgelenkpfanne (1) nach Anspruch 3, dadurch gekennzeichent, dass der Rand der kreisförmigen Ausnehmung (4g) Zähne (4f) aufweist, die in den Innenraum der kreisförmigen Ausnehmung (4g) vorstehen.

5. Künstliche Hüftgelenkpfanne (1) nach einem der Ansprüche 1 bis 4, dadurch gekennzeichnet, dass mindestens eines der Befestigungsmittel (5) als Einrastelement (4e) ausgebildet ist, wobei die Stützvorrichtung (2) eine Durchbrechung (2f) aufweist, durch die das Einrastelement (4e) durchführbar ist, um im Austrittsbereich der Durchbrechung (2f) einzurasten.

6. Künstliche Hüftgelenkpfanne (1) nach einem der Ansprüche 1 bis 5, dadurch gekennzeichnet, dass die Stützschale (2d) eine Innenfläche (2o) aufweist, aus der mindestens ein Eindringelement (2e) vorsteht.

7. Künstliche Hüftgelenkpfanne (1) nach einem der Ansprüche 1 bis 6, dadurch gekennzeichnet, dass die Innenschale (3) aus Kunststoff, Metall oder Porzellan besteht.

8. Verfahren zum Zusammenstellen einer Hüftgelenkpfanne (1) gemäss einem der Ansprüche 1 bis 7, dadurch gekennzeichnet,
- dass die Haltevorrichtung (4) mit der Stützvorrichtung (2) so verbunden wird, dass das Abstützelement (4b) der Haltevorrichtung und die Auflage (2c) der Stützvorrichtung (2) ein Widerlager bilden,
- dass die Innenschale (3) in die Stützschale (2d) eingeführt und die gegenseitige Lage von Innenschale (3) und Stützschale (2d) festgelegt wird,
- und dass die Haltevorrichtung (4) um eine durch das Widerlager definierte Achse geschwenkt und mit den Befestigungsmitteln (5) an der Stützvorrichtung (2) befestigt wird, sodass die Innenschale (3) zwischen der Stützschale (2d) und der Haltevorrichtung (4) eingespannt wird.

## Claims

1. Artificial hip joint socket (1) comprising:
- a metallic support device (2) comprising a support shell (2d) with an equatorial edge (2p) and support lugs (2a) projecting outwardly from the equatorial edge (2p), wherein the support lugs (2a) have apertures (2b) for bone screws,
- an inner shell (3) for receiving a head of a ball joint and fitted into the support shell (2d),
- a holding device (4) and at least two securing means (5), wherein the holding device (4) is securely connected to the support device (2) via the securing means (5) in order to clamp the inner shell (3) between the support shell (2d) and the holding device (4).
characterized in that the holding device (4) comprises a support element (4b), and in that the support device (2) comprises a mount (2c), so that in case of an effective connection between the support element (4b) and the mount (2c) an abutment results.

2. Artificial hip joint socket (1) in accordance with claim 1, characterized in that the holding device (4) has two symmetrically arranged support elements (4b), which basically surround a lug (2a) on both sides, thus forming a fixation means (5).

3. Artificial hip joint socket (1) in accordance with one of the claims 1 or 2, characterized in that the inner shell (3) has an outer surface (3d) which is ball shaped, in that the holding device (4) has a circular recess (4g), and in that the edge of the circular recess (4g) lies on the outer surface (3d) when the holding device (4) is secured to the support device (2).

4. Artificial hip joint socket (1) in accordance with claim 3, characterized in that the edge of the circular recess (4g) has teeth (4f) which protrude into the inner space of the circular recess (4g).

5. Artificial hip joint socket (1) in accordance with one of the claims 1 to 4, characterized in that at least one of the securing means (5) is formed as a latch element (4e), wherein the support device (2) has an aperture (2f) through which the latch element (4e) reaches in order to latch into place in the exit region of the aperture (2f).

6. Artificial hip joint socket (1) in accordance with one of the claims 1 to 5, characterized in that the support shell (2d) has an inner surface (20), from which at least one penetration element (2e) projects.

7. Artificial hip joint socket (1) in accordance with one of the claims 1 to 8, characterized in that the inner shell (3) is made of plastic, metal or porcelain.

8. Method for assembling a hip joint socket (1) in accordance with one of the claims 1 to 7, characterized in that
- the holding device (4) is connected to the support device (2) in such a way that the support element (4b) of the holding device and the supporting portion (2c) of the support device (2) form an abutment
- in that the inner shell (3) is introduced into the support shell (2d) and the mutual position of inner shell (3) and support shell (2d) is fixed,
- and in that the holding device (4) is pivoted around an axis defined by the abutment and is fixed to the support device (2) by means of securing means (5), so that the inner shell (3) is clamped between the support shell (2d) and the holding device (4).

## Revendications

1. Coque acétabulaire artificielle (1), comprenant
- un dispositif d'appui métallique (2) qui est constitué d'une coque d'appui (2d) avec un bord équatorial (2p) ainsi que de languettes d'appui (2a) faisant saillie du bord équatorial (2p) vers l'extérieur, qui présentent des perçages (2b) pour des vis à os,
- une coque intérieure (3) pour la réception d'une tête sphérique d'articulation qui est placée dans la coque d'appui (2d),
- un dispositif de retenue (4) et au moins deux moyens de fixation (5), le dispositif de retenue (4) pouvant être assemblé par les moyens de fixation (5) solidement avec le dispositif d'appui (2) pour mettre en tension la coque intérieure (3) entre la coque d'appui (2d) et le dispositif de retenue (4), caractérisée en ce que le dispositif de retenue (4) comprend un élément d'appui (4b) et que le dispositif d'appui (2) présente un appui (2c) de façon à obtenir lors d'une liaison active entre l'élément d'appui (4b) et l'appui (2c) une butée.

2. Coque acétabulaire artificielle (1) selon la revendication 1, caractérisée en ce que le dispositif de retenue (4) présente deux éléments d'appui (4b) disposés symétriquement qui entourent essentiellement une languette (2a) des deux côtés et forment ainsi un moyen de fixation (5).

3. Coque acétabulaire artificielle (1) selon l'une des revendications 1 ou 2, caractérisée en ce que la coque intérieure (3) présente une face extérieure (3d) qui est réalisée en forme sphérique, que le dispositif de retenue (4) présente un évidement circulaire (4g) et que, lorsque le dispositif de retenue (4) est fixé au dispositif d'appui (2), le bord de l'évidement circulaire (4g) repose sur la face extérieure (3d).

4. Coque acétabulaire artificielle (1) selon la revendication 3, caractérisé en ce que le bord de l'évidement circulaire (4g) présente des dents (4f) qui font salle dans l'espace intérieur de l'évidement circulaire (4g).

5. Coque acétabulaire artificielle (1) selon l'une des revendications 1 à 4, caractérisée en ce qu'au moins l'un des moyens de fixation (5) est réalisé comme élément d'enclenchement (4e), où le dispositif d'appui (2) présente un perçage (2f) à travers lequel peut passer l'élément d'enclenchement (4e) pour s'enclencher dans la zone de sortie du perçage (2f).

6. Coque acétabulaire artificielle (1) selon l'une des revendications 1 à 5, caractérisée en ce que la coque d'appui (2d) présente une face intérieure (20) de laquelle dépasse au moins un élément de pénétration (2e).

7. Coque acétabulaire artificielle (1) selon l'une des revendications 1 à 6, caractérisée en ce que la coque intérieure (3) est réalisée en matière synthétique, métal ou porcelaine.

8. Procédé d'assemblage d'une coque acétabulaire (1) selon l'une des revendications 1 à 7, caractérisé en ce
- que le dispositif de retenue (4) est relié au dispositif d'appui (2) de façon que l'élément d'appui (4b) du dispositif de retenue et l'appui (2c) du dispositif d'appui (2) forment une butée,
- que la coque intérieure (3) est insérée dans la coque d'appui (2d) et que la position mutuelle de la coque intérieure (3) et de la coque d'appui (2d) est fixée,
- et que le dispositif de retenue (4) est amené à pivoter autour d'un axe défini par la butée et est fixé avec les moyens de fixation (5) au dispositif d'appui (2) de telle sorte que la coque intérieure (3) est encastrée entre la coque d'appui (2d) et le dispositif de retenue (4).
